Europäisches Patentamt

European Patent Office

Office européen des brevets

(11) Veröffentlichungsnummer: **0 511 538 A2**

# EUROPÄISCHE PATENTANMELDUNG

(21) Anmeldenummer: **92106377.2**

(51) Int. Cl.5: **A61M 39/00**

(22) Anmeldetag: **14.04.92**

(30) Priorität: **27.04.91 DE 9105229 U**

(43) Veröffentlichungstag der Anmeldung:
**04.11.92 Patentblatt 92/45**

(84) Benannte Vertragsstaaten:
**AT BE CH DE DK ES FR GB GR IT LI NL SE**

(71) Anmelder: **B. Braun Melsungen AG**
**Carl-Braun Strasse**
**W-3508 Melsungen(DE)**

(72) Erfinder: **Schneider, Stefan**
**Immelmannstrasse 15**
**W-2300 Kiel 17(DE)**
Erfinder: **Maier, Hans-Otto**
**Am Sandberg 46**
**W-3503 Lohfelden(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

(54) **Ventilvorrichtung für einen Katheter.**

(57) Die Ventilvorrichtung für einen Katheter mit Katheteransatz besteht aus einem rohrförmigen Gehäuse (11), das an seinem ersten Ende einen Außenkonus (13) zur Einpassung in einen Innenkonus des Katheteransatzes (30) aufweist und das im Inneren einen Ventilkörper als Absperrorgan eines axialen Durchganges zum Durchlaß eines langgestreckten Gegenstandes enthält. Erfindungsgemäß ist vorgesehen, daß das Gehäuse (11) mit einem axial gerichteten Fortsatz versehen ist, der mit der Außenfläche des Außenkonus (13) einen radialen Zwischenraum (14) bildet und der ein Verriegelungsorgan aufweist, welches mit einem Gegenorgan an dem in den Zwischenraum (14) hineinragenden Katheteransatz (30) als axiale Dekonnektierungssperre zusammengreift. Auf diese Weise wird eine Ventilvorrichtung geschaffen, die geeignet ist für ein Kathetersystem zum venösen Einsatz nach der Seldinger-Methode und die einen zuverlässigen Verschluß des extrakorporalen Endes des Katheters gewährleistet.

FIG.1

Rank Xerox (UK) Business Services

Die Erfindung bezieht sich auf eine Ventilvorrichtung nach dem Oberbegriff des Anspruches 1.

Eine aus DE 30 42 229 C2 bekannte Ventilvorrichtung der erwähnten Art dient zum Einbringen langgestreckter Gegenstände durch eine Kanüle in ein Blutgefäß und ihr Außenkonus wird mit dem Innenkonus des Ansatzes klemmend zusammengesteckt. Der Ventilkörper besteht hierbei aus einer Aufeinanderfolge von geschlitzten Dichtungselementen, deren Dichtlippen sich an die langgestreckten Gegenstände bzw. gegeneinander anlegen und die Passagen verschließen. Diese Ventilvorrichtung kann nur als Schleuse für von außen in das Blutgefäß eingeführte Führungsdrähte oder dergleichen benutzt werden. Sie ist nicht geeignet für ein Kathetersystem zum venösen Einsatz nach der Seldinger-Methode, bei der der Katheter mit seiner Spitze über einen verlegten Führungsdraht gefädelt wird. Dies ist darauf zurückzuführen, daß die zwischen Ventilkörper und Führungsdraht entstehenden Reibungskräfte das "feeling" für das Verlegen des Katheters stark beeinflußen, so daß das Gefühl für das Verlegen bis zu einem gewissen Grade verlorengeht. Ferner ist es bei dieser Ventilvorrichtung praktisch unmöglich, beim Auffädeln eines Katheters auf den Führungsdraht mit dessen Spitze in den Schlitz der Dichtungselemente zu treffen. Außerdem ist diese bekannte Ventilvorrichtung nicht zum absolut sicheren abdichtenden Verschluß des Durchganges eines Katheters nach Entfernung des Führungsdrahtes aus seinem Lumen geeignet, weil die Steckverbindung zwischen Außenkonus und Innenkonus des Katheteransatzes nicht zuverlässig haltbar ist. Es besteht die Gefahr, daß sich die Steckverbindung bei länger liegendem Katheter durch Bewegungen des Patienten lockert mit der Folge, daß Luft in den Katheter eindringen kann, die bei Verlegung des Katheters in der Hohlvene eines Patienten zum Tode führen kann. Bei weniger kritischer Anwendung wird durch die Steckverbindungslockerung eine Leckstelle geschaffen, durch die rückströmende Körperflüssigkeit austreten kann, die aus der gelockerten Verbindungsstelle leckt und Kontaminationen des Umfeldes hervorruft, die für den Patienten und das Pflegepersonal gefährlich sein können.

Bei einer anderen bekannten Ventilvorrichtung nach DE 28 17 102 C2 ergeben sich ähnliche Verhältnisse, die ihre Verwendung für ein Kathetersystem zum venösen Einsatz nach der Seldinger-Methode ausschließt. Es ist ein begrenzt axial verschiebbarer Körper in einem Durchflußkanal eines rohrförmigen Gehäuses untergebracht, der mit einer den Durchflußkanal absperrenden Scheibe aus elastomerem Material mit einem zentralen Schlitz zusammenwirkt. Der Körper ist als Hülse mit kegelstumpfförmig verjüngtem Ende ausgebildet. Seine Verschiebung ergibt sich bei Ansetzen eines Anschlußkonus. Dabei dringt das kegelstumpfförmige Ende in den Schlitz der Scheibe ein, spreizt ihn und hält ihn offen. Dadurch wird die Reibung zwischen den Schlitzlippen und einem von außen eingeschobenen langgestreckten Element vermindert. Zur Ausnutzung dieses Vorteils beim Auffädeln eines mit dieser Ventilvorrichtung ausgerüsteten Venenkatheters auf das Ende eines verlegten Führungsdrahtes, müßte der Körper durch einen Anschlußkonus zur Öffnung des Schlitzes verschoben werden, dieser Anschlußkonus allerdings würde seinerseits die Verlegung des Katheters erschweren. Das Gehäuse dieser Ventilvorrichtung ist an einem Ende mit einem Innenkonus für den Anschlußkonus zur Betätigung des Körpers versehen und weist am anderen Ende einen konusförmig verjüngten Stutzen mit umlaufenden Rippen auf, der der Halterung eines zu dem Gefäßsystem führenden aufgeschobenen Schlauches ohne Ansatzstück dient. Eine solche Verbindung zwischen Schlauch und Ventilvorrichtungsgehäuse ist unzuverlässig. Würde die Ventilvorrichtung in eine Leitungsanordnung eingesetzt, könnte sich die Verbindung durch Bewegungen lösen und es ergäbe sich durch Lecks ausströmender oder einströmender Medien eine Gefährdung des Patienten und/oder des Personals.

Der Erfindung liegt die Aufgabe zugrunde, eine Ventilvorrichtung zu schaffen, die geeignet ist für ein Kathetersystem zum venösen Einsatz nach der Seldinger-Methode und die einen zuverlässigen Verschluß des extrakorporalen Endes des Katheters gewährleistet.

Diese Aufgabe wird erfindungsgemäß dadurch gelöst, daß das Gehäuse mit einem axial gerichteten Fortsatz versehen ist, der mit der Außenfläche des Außenkonus einen radialen Zwischenraum bildet und der ein Verriegelungsorgan aufweist, welches mit einem Gegenorgan an dem in den Zwischenraum hineinragenden Katheteransatz als axiale Dekonnektierungssperre zusammengreift.

Die Erfindung nutzt die Erkenntnis aus, daß das "feeling" für die Zuverlässigkeit der Verlegung eines Katheters nach der Seldinger-Methode bedeutungsvoller als die Tatsache ist, daß beim Verlegen des Katheters durch Aufstecken auf den Führungsdraht etwas Blut austritt, denn der Blutkontakt ist sowieso bei dieser Verlegungsart nicht zu vermeiden.

Die erfindungsgemäße Ventilvorrichtung wird erst nachträglich mit einem durch Aufschieben auf einen verlegten Führungsdraht in die Vene eingeführten Katheter verbunden, so daß der Katheter nach der Seldinger-Methode ohne Ventilvorrichtung unter vollständiger Erhaltung des "feeling" verlegt wird. Erst nach erfolgreicher Katheterverlegung und Herausziehen des Führungsdrahtes aus dem Katheter wird die Ventilvorrichtung an dem Katheteransatz nicht dekonnektierbar montiert, damit Flüssigkeitsrücktritt und Lufteinschleppung verhindert werden. Die Ventilvorrichtung wird einem Katheterset mit Führungsdraht separat beigefügt und erst vom Anwender nach der Katheterverlegung so angesetzt, daß sie sich von diesem nicht mehr abnehmen läßt. Das in einfacher Weise mit einem Katheteransatz unlösbar kuppelbare Ventilgehäuse ist mit dem Katheteransatz verriegelt, so daß es sich bei Bewegungen des Patienten nicht von dem Katheteransatz lösen kann. Die extrakorporale Öffnung des Katheters bleibt stets zuverlässig mit der Ventilvorrichtung verbunden, so daß Kontaminationen des Umfeldes durch Körperfluidaustritt und eine Gefährdung des Patienten durch in den Katheter eindringende Luft verhindert werden. Die Sicherheit eines katheterisierten, an ein Überleitungssystem angeschlossenen mobilen Patienten wird durch die erfindungsgemäße Ventilvorrichtung erhöht. Die Ventilvorrichtung gewährleistet außerdem für das Wechseln eines im Gefäß liegenden Katheters, daß durch den Ventilkörper von außen ein Führungsdraht gesteckt werden kann, über den man den zu entfernenden Katheter aus dem Gefäß zieht. Das Verlegen des neuen ventillosen Katheters kann dann wie gewöhnlich über den Führungsdraht erfolgen. Liegt der Katheter in der gewünschten Position, wird wiederum die nicht dekonnektierbare erfindungsgemäße Ventilvorrichtung montiert.

An dem zweiten Ende des Gehäuses ist vorteilhafterweise ein Anschlußprofil für einen Konnektor ausgebildet. Das Anschlußprofil ist vorzugsweise Luer-Lock-konform und ermöglicht den Anschluß einer weiterführenden Leitung oder eines Betätigungsorganes für den Ventilkörper. Eine weiterführende Leitung kann z.B. zu einem Überleitungssystem gehören. Die Verriegelung des Ventilvorrichtungsgehäuses mit dem Katheteransatz sorgt für zuverlässige Dichtigkeit des Anschlusses. Die Ausbildung des Ventilkörpers kann in vielfältiger Weise vorgesehen sein - unter der Voraussetzung, daß er einen abgedichteten Durchlaß für einen langgestreckten Gegenstand schafft, der sich nach Herausziehen des Gegenstandes wieder dicht verschließt.

In vorteilhafter Ausgestaltung der Erfindung ist der Fortsatz als kreiszylindrische Hülse mit einwärts gerichteter Ringwulst ausgebildet, die von radialen Außenvorsprüngen des Katheteransatzes nach Art einer Ringschnappverbindung hintergriffen ist. Die kreiszylindrische Hülse als zylindrisches Schnappelement ist zur Verbindung mit herkömmlichen Katheteransätzen geeignet, die zwei diametral gegenüberliegende, nach außen gerichtete radiale Verriegelungsnocken haben. Die beim Einschnappvorgang auftretende Auslenkung, die man als Hinterschnitt bezeichnet, muß so bemessen sein, daß die zulässige Dehnung der verwendeten Werkstoffe nicht überschritten wird und nach dem Einrasten eine entspannte formschlüssige Verbindung vorliegt, die sich nicht axial auseinanderziehen läßt. Die Ringschnappverbindung dient dazu, die beiden miteinander verbundenen Bauteile in einer bestimmten Lage zueinander zu fixieren. Ihr Vorteil ist es, daß sie sich mit geringer Fügekraft leicht montieren läßt. Die unlösbare Ringschnappverbindung hält den Außenkonus des Gehäuses in dem Innenkonus des Katheteransatzes fest und garantiert damit die Dichtigkeit der Verbindung.

Der Mantel der kreiszylindrischen Hülse kann geschlossen sein oder Schnappsegmente bildende Längsschlitze aufweisen. Im ersten Falle ist die Fügekraft bei der Montage größer als im zweiten Falle und an die elastische Verformbarkeit der Kunststoffe beider Bauteile werden erhöhte Anforderungen gestellt. In beiden Fällen ist es wichtig, daß nach kurzzeitiger Verformung beim Fügeprozeß der Werkstoff sich rasch wieder zurückstellt, damit der Zusammengriff von Verriegelungsorgan und Gegenorgan als axiale Dekonnektierungssperre wirksam ist. Bei Längsschlitzung der kreiszylindrischen Hülse ist eine Viertelteilung oder Halbierung des kreiszylindrischen Mantels für eine nichtlösbare Schnappverbindung günstig.

Die Ringwulst kann etwa halbkreisförmiges Querschnittsprofil haben. Alternativ ist es möglich, ihr etwa trapezförmiges Querschnittsprofil zu geben, so daß sich auf einer Seite eine Gleitfläche zur Erleichterung des Fügevorganges und auf der anderen Seite eine Sperrfläche ergeben. Beide Querschnittsprofile sind für unlösbare Schnappverbindungen günstig.

In weiterer vorteilhafter Ausgestaltung der Erfindung ist vorgesehen, daß der Fortsatz aus mindestens einem teilkreiszylindrischen Flügel mit einer Nut gebildet ist, die von einer randseitigen Öffnung zu einem Umfangsabschnitt mit geschlossenem Ende verläuft und in die ein radialer Stift an der Außenfläche des Katheteransatzes nach Art einer Bajonettverbindung einrastend eingeführt ist.

In diesem Falle ist die Dekonnektierungssperre aus einem in eine Nut eingreifenden Stift gebildet, der an dem Katheteransatz angebracht ist. Die Nut, die auf der Außenfläche des teilkreiszylindrischen Flügels offen oder geschlossen sein kann, verläuft entweder L-förmig oder in Umfangsrichtung angenähert S-förmig und vor ihrem inneren Ende ist ein Rastvorsprung ausgebildet, den der Stift einrastend hintergreift. In beiden Fällen wird der Stift durch Relativdrehung von Katheteransatz und Ventilvorrichtungsgehäuse gegen das innere Ende der Nut zur Anlage gebracht und eingerastet, so daß sich eine axiale Dekonnektierungssperre ergibt.

Das Anschlußprofil an dem zweiten Ende des Gehäuses besteht vorzugsweise aus einem Innenkonus in einem Rohrstutzen, der außen von einem Gewindeteil umgeben ist. Diese Ausbildung ist Luer-Lock-konform, d.h., das Anschlußprofil läßt sich mit einem verriegelbaren Konnektor mit Außenkonus verbinden, so daß z.B. eine Leitung anschließbar ist und auch an diesem Ende der Ventilvorrichtung für sichere Verbindung innerhalb eines Leitungsstranges gesorgt ist. Vorzugsweise ist der Ventilkörper ein gummielastischer becherförmiger Hohlzylinder, der von dem Außenkonus des Konnektors bzw. einer ihn umgebenden Schraubenfeder betätigt wird, wie in Anspruch 8 angegeben ist. Sobald der Konnektor gelöst und sein Außenkonus von dem Ventilvorrichtungsgehäuse abgezogen wird, schließt der Ventilkörper durch Federwirkung dicht und es kann weder Luft in den Katheter einströmen, noch Körperfluid aus diesem austreten. Der Ventilkörperdurchlaß ist wieder öffenbar, wenn ein Führungsdraht eingeführt werden soll, über den man den verlegten Katheter aus dem Gefäß herausziehen und einen neuen Katheter ohne Ventilvorrichtung einführen kann.

In der Zeichnung sind Ausführungsbeispiele der Erfindung schematisch dargestellt. Es zeigt:

Fig. 1 einen Längsschnitt durch die Ventilvorrichtung mit einer ersten Ausführungsform einer Ringschnappverbindung,

Fig. 2 eine Seitenansicht zur Veranschaulichung der Ringschnappverbindung nach Figur 1,

Fig. 3 einen Schnitt längs der Linie III-III in Figur 1,

Fig. 4 einen Längsschnitt durch eine Ventilvorrichtung mit einer zweiten Ausführungsform einer Ringschnappverbindung,

Fig. 5 eine Seitenansicht einer als Bajonettverbindung ausgebildeten Dekonnektierungssperre, und

Fig. 6 eine Draufsicht auf die Anordnung in der Position nach Figur 5.

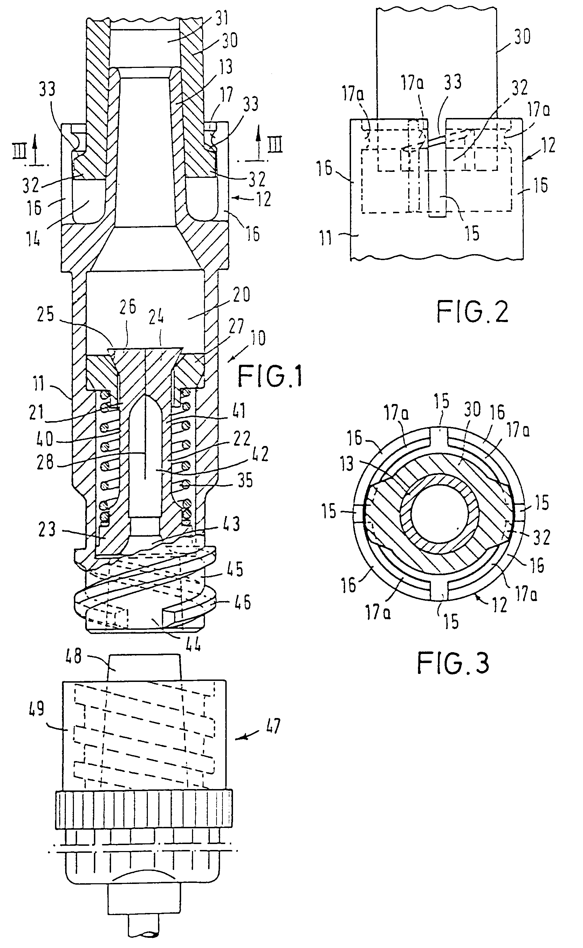

Die Ventilvorrichtung 10 gemäß Figuren 1 bis 3 besteht aus einem als Spritzgußteil gefertigten rohrförmigen Gehäuse 11 aus Kunststoffmaterial. An das erste Ende des Gehäuses 11 ist eine koaxiale kreiszylindrische Hülse 12 angespritzt, die einen Außenkonus 13 etwas größerer Länge mit gleichmäßigem radialen Abstand umgibt, so daß sich ein Zwischenraum 14 zwischen der Außenfläche des Außenkonus 13 und der Innenfläche der kreiszylindrischen Hülse 12 bildet. Die Hülse 12 und der Außenkonus 13 sind koaxial angeordnet und liegen auf der Längsachse des rohrförmigen Gehäuses 11. Im äußeren Randbereich der Hülse 12 ist eine radial einwärts gerichtete Ringwulst 17 vorgesehen, die halbkreisförmiges Querschnittsprofil hat und aus vier gleichen Kreisabschnitten 17a zusammengesetzt ist. Die Kreisabschnitte 17a der Ringwulst 17 ergeben sich dadurch, daß die kreiszylindrische Hülse 12 durch vier symmetrische Längsschlitze 15 in vier Schnappsegmente 16 unterteilt ist. Die Schnappsegmente 16 bilden eine axiale Dekonnektierungssperre für einen Katheteransatz 30 eines vorzugsweise langen nicht gezeichneten Venenkatheters. Auch der Katheteransatz 30 besteht aus Kunststoff. Er weist einen Innenkonus 31 auf, in den der Außenkonus 13 passend und dicht einfügbar ist. Am äußeren Rand des Katheteransatzes 30 befinden sich zwei diametral gerichtete radiale Außenvorsprünge 32, die in üblicher Weise als Verriegelungsnocken mit schräger Unterfläche 33 gestaltet sind.

In dem kreiszylindrischen Hohlraum 20 des rohrförmigen Gehäuses 11 ist ein gummielastischer becherförmiger Hohlzylinder 21 koaxial angeordnet, der als Ventilkörper dient. Der Hohlzylinder 21 weist einen kreiszylindrischen Schaft 22 auf, der an einem Ende einen äußeren Profilkranz 23 und am anderen Ende einen axial dicken Bodenteil 24 hat, der an seinem freien Ende mit einem divergierenden äußeren Ringkonus 25 versehen ist. Seine äußere Bodenfläche 26 ist kreisförmig und eben. Ein Schließring 27 mit einer der Konizität 25 des Bodenteils 24 angepaßten Abschrägung umgibt den Bodenteil 24 in Schließposition des Hohlzylinders 21. Der dicke Bodenteil 24 ist durch einen ihn diametral durchschneidenden Längsschlitz 28, der sich bis weit in den Schaft 22 hineinerstreckt, in zwei Schenkel 40, 41 aufgeteilt, die sich bis zum inneren Ende des Längsschlitzes 28 auseinanderspreizen können, wenn sie von dem Schließring 27 freigekommen sind. Die Wandung des Hohlzylinders 21 umschließt einen kreiszylindrischen Längskanal 42, der einerseits an der inneren Grundfläche des Bodenteiles 24 in einer Kegelvertiefung endet und andererseits durch eine Öffnung 43 mit einem Innenkonus 44 eines Rohrstutzens 45 offen in Verbindung steht, der auf seiner Außenfläche von einem Gewindeteil 46 umgeben ist. Auf den Gewindeteil 46 ist eine Schraubkappe 49 eines Konnektors 47 aufschraubbar, dessen Außenkonus 48 in den Innenkonus 44 abdichtend hineinragt.

Der Hohlzylinder 21 ist von einer Schraubenfeder 35 umgeben, deren eines Ende sich an einer Schulter des äußeren Profilkranzes 23 des Hohlzylinders 21 abstützt und deren anderes Ende gegen eine Ringfläche des Schließringes 27 drückt. Die Schraubenfeder 40 ist vorgespannt und hält den Hohlzylinder 21 in Schließposition, in der sein Bodenteil 24 in die konische Vertiefung des Schließringes 27 hineingezogen ist und der radial äußere Teil des Randes seiner Öffnung 43 gegen eine Schulter am inneren Ende des Innenkonus 44 anliegt. Wenn der Konnektor 47 an den Rohrstutzen 46 angesetzt ist, wird der Hohlzylinder 21 von der Stirnfläche des Außenkonus 48 axial in den Hohlraum 20 hineingedrückt und die beiden Schenkel 40, 41 spreizen sich unter dem Druck einströmender Flüssigkeit auseinander.

Wenn ein ventilloser Katheter nach der Seldinger-Methode mit Hilfe eines Führungsdrahtes verlegt und dieser herausgezogen worden ist, wird der Katheteransatz 30 mit der Ventilvorrichtung 10 zusammengefügt. Dies geschieht durch axiales Aufschieben der Hülse 12 auf den Katheteransatz 30. Dabei werden die Schnappsegmente 16 von den beiden radialen Außenvorsprüngen 32 radial ausgelenkt und der Außenkonus 13 dringt in den Innenkonus 31 ein. Sobald die Außenvorsprünge 32 die Ringwulst 17 passiert haben, stellen sich die Schnappsegmente 16 durch die Elastizität des Materials wieder zurück und die Außenvorsprünge 32 hintergreifen die Ringwulst 17. Diese Ringschnappverbindung bildet eine Dekonnektierungssperre, die eine Trennung von Außenkonus 13 und Innenkonus 31 durch axiales Abziehen verhindert, so daß eine dichte dauerhafte Verbindung zwischen dem Katheteransatz 30 und der Ventilvorrichtung 10 gewährleistet ist. Wenn der in dem Gefäß liegende Katheter gewechselt werden soll, wird der Konnektor 47 von dem Rohrstutzen 45 der Ventilvorrichtung 10 abgenommen und es wird in den Innenkonus 44 ein Betätigungsteil eingesteckt, der den Hohlzylinder 21 in Öffnungsrichtung verschiebt. Durch den Hohlzylinder 21 wird ein Führungsdraht gesteckt, über den der Katheter mit der Ventilvorrichtung 10 aus dem Gefäß gezogen wird. Auf den verlegten Führungsdraht wird nun ein neuer ventilloser Katheter in üblicher Weise nach der Seldinger-Methode aufgefädelt. Liegt der neue Katheter in der gewünschten Position, wird eine neue Ventilvorrichtung 10 mit seinem Katheteransatz 30 nicht dekonnektierbar verbunden.

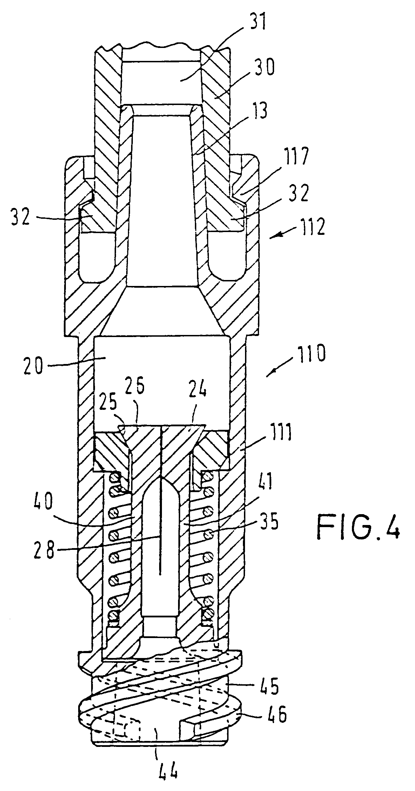

Die Ventilvorrichtung 110 nach Figur 4 entspricht hinsichtlich des Ventilkörpers und des Anschlußprofils am zweiten Ende des Gehäuses 111 dem Beispiel nach Figuren 1 bis 3. Lediglich die Dekonnektierungssperre ist etwas anders gestaltet. Die Ringschnappverbindung wird in diesem Falle aus einer kreiszylindrischen Hülse 112 mit ringsum geschlossenem Mantel gebildet. Am äußeren Randbereich der Hülse 112 ist innen mit geringfügigem Abstand zur Außenkante eine Ringwulst 117 vorgesehen, die sich unterbrechungslos über den gesamten Umfang der Hülse 112 erstreckt und die ein etwa trapezförmiges Querschnittsprofil hat. Während des Fügevorganges wird die Hülse 112 radial aufgeweitet und stellt sich wieder in ihre kreiszylindrische Ursprungsform zurück, sobald die Außenvorsprünge 32 des Katheteransatzes 30 hinter die Ringwulst 117 greifen und der Einschnappvorgang beendet ist. Es wird eine nicht lösbare Schnappverbindung erreicht, bei der der Hinterschnitt so bemessen ist, daß die für das Kunststoffmaterial zulässige Dehnung nicht überschritten wird. Die Abschrägungen der Ringwulst 117 sind für den Fügevorgang günstig. Auch haben sie den Vorteil guter Entformbarkeit bei der Herstellung. Die Schnappverbindung sorgt für eine spielfreie Zusammenhaltung, wenn der Zusammengriff von Ringwulst 117 und Außenvorsprüngen 32 in einem solchen axialen Abstand von der Stirnkante des Außenkonus 13 erfolgt, daß die Eindringtiefe des Außenkonus 13 in den Innenkonus 31 für den abgedichteten Verschluß des Katheterlumens ausreicht.

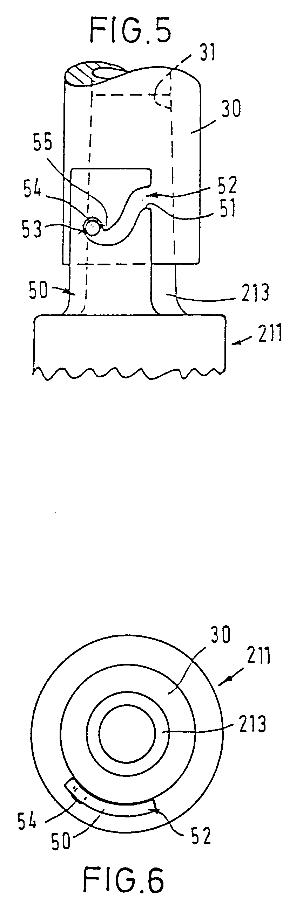

In den Figuren 5 und 6 ist ein nur angedeutetes Gehäuse 211 einer Ventilvorrichtung mit einem Außenkonus 213 ausgestattet, der koaxial von der Stirnseite des Gehäuses 211 absteht. Als Dekonnektierungssperre dient ein teilkreiszylindrischer Flügel 50, der mit radialem Abstand von dem Außenkonus 213 an dem Gehäuse 211 vorgesehen ist. In dem Flügel 50 ist eine Nut 51 ausgebildet, die die Wand des Flügels 50 nach Art eines Schlitzes durchbricht. Die Nut 51 ist an einem Flügel-Längsrand bei 52 offen und verläuft mit umfangsmäßiger Steigung leicht S-förmig gebogen bis zu ihrem geschlossenen Ende 53, das eine nach oben weisende Tasche bildet. In der Nut 51 ist am Beginn der Tasche ein Rastvorsprung 55 angeordnet, der den Durchlaß der Nut verengt. Der Flügel 50 ist axial kürzer als der Außenkonus 213. In die Nut 51 wird beim Zusammenstecken von Innenkonus 31 und Außenkonus 213 durch Drehung ein radialer Stift 54 hineinbewegt, der von der Außenfläche des Katheteransatzes 30 radial absteht. Der in die Nut 51 eindringende Stift 54 bildet mit dem Flügel 50 eine Art Bajonettverbindung, bei der in Arretierposition der Stift 54 den Rastvorsprung 55 hintergreift, so daß er in der Tasche am geschlossenen Ende 53 der Nut 51 gefangen ist. In diesem gegen Dekonnektierung gesperrten Zustand ist der Außenkonus 213 fest in den Innenkonus 31 hineingezogen, so daß die Verbindung dicht ist. Bewegungen des Leitungsstranges, in den die Ventilvorrichtung eingebaut ist, können die Verbindung nicht lockern und ihr Zusammenhalt bei axialer Krafteinwirkung ist gewährleistet. Bewegungen des Leitungsstranges zwischen einem Überleitungssystem und dem Patienten liegen insbesondere bei der zunehmend geübten frühen Mobilisierung der Patienten, die das Überleitungssystem beim freien Umhergehen mit sich tragen, vor.

**Patentansprüche**

1. Ventilvorrichtung für einen Katheter mit Katheteransatz, bestehend aus einem rohrförmigen Gehäuse, das an seinem ersten Ende einen Außenkonus zur Einpassung in einen Innenkonus des Katheteransatzes aufweist und das im Inneren einen Ventilkörper als Absperrorgan eines axialen Durchganges zum Durchlaß eines langgestreckten Gegenstandes enthält, **dadurch gekennzeichnet,** daß

das Gehäuse (11) mit einem axial gerichteten Fortsatz versehen ist, der mit der Außenfläche des Außenkonus (13) einen radialen Zwischenraum (14) bildet und der ein Verriegelungsorgan aufweist, welches mit einem Gegenorgan an dem in den Zwischenraum (14) hineinragenden Katheteransatz (30) als axiale Dekonnektierungssperre zusammengreift.

2. Ventilvorrichtung nach Anspruch 1, **dadurch gekennzeichnet,** daß

an dem zweiten Ende des Gehäuses (11) ein Anschlußprofil für einen Konnektor ausgebildet ist.

3. Gehäuse nach Anspruch 1 oder 2, **dadurch gekennzeichnet,** daß

der Fortsatz als kreiszylindrische Hülse (12;112) mit einwärts gerichteter Ringwulst (17;117) ausgebildet ist, die von radialen Außenvorsprüngen (32) des Katheteransatzes (30) nach Art einer Ringschnappverbindung hintergriffen ist .

4. Gehäuse nach Anspruch 3, **dadurch gekennzeichnet,** daß

der Mantel der kreiszylindrischen Hülse (12;112) geschlossen ist oder Schnappsegmente (16) bildende Längsschlitze (15) aufweist.

5. Gehäuse nach Anspruch 3, **dadurch gekennzeichnet,** daß

die Ringwulst (17) etwa halbkreisförmiges Querschnittsprofil hat.

6. Gehäuse nach Anspruch 3, **dadurch gekennzeichnet,** daß

die Ringwulst (117) etwa trapezförmiges Querschnittsprofil hat.

7. Gehäuse nach Anspruch 1, **dadurch gekennzeichnet,** daß

der Fortsatz aus mindestens einem teilkreiszylindrischen Flügel (50) mit einer Nut (51) gebildet ist, die von einer randseitigen Öffnung (52) zu einem Umfangsabschnitt mit geschlossenem Ende (53) verläuft

und daß in die Nut (51) ein radialer Stift (54) an der Außenfläche des Katheteransatzes (30) nach Art einer Bajonettverbindung einrastend eingeführt ist.

8. Gehäuse nach einem der Ansprüche 1 bis 7, **dadurch gekennzeichnet,** daß

das Anschlußprofil an dem zweiten Ende des Gehäuses (11) aus einem Innenkonus (44) in einem Rohrstutzen (45) besteht, der außen von einem Gewindeteil (46) umgeben ist, daß ein gummielastischer becherförmiger Hohlzylinder (21) mit axial geschlitztem Bodenteil (24) in einem Hohlraum (20) am zweiten Ende des Gehäuses (11) axial beweglich angeordnet ist, daß der Hohlzylinder (21) von einer ihn umgebenden Feder (35) in Schließstellung gehalten ist, in der der radial äußere Teil seines Öffnungsrandes gegen eine Schulter am inneren Ende des Innenkonus (44) angedrückt ist und der radial innere Teil seines Öffnungsrandes in den Querschnitt des Innenkonus (44) hineinragt und daß der Bodenteil (24) in eine angepaßte zentrale Lochöffnung eines in dem Gehäuse (11) fixierten Schließringes (27) hineingezogen ist, aus der der Bodenteil (24) zur Freigabe seiner durch die Schlitzung (28) gebildeten Schenkel (40,41) gegen die Federkraft axial herausdrückbar ist.

FIG.1

FIG.2

FIG.3

FIG.4

## FIG.5

## FIG.6